# EUROPEAN PATENT SPECIFICATION

(11) **EP 1 870 048 B1**
(45) Date of publication and mention of the grant of the patent: **11.08.2010**
(21) Application number: 07252340.0
(22) Date of filing: 08.06.2007
(51) Int. Cl.: A61B 17/68, A61B 17/80, A61B 19/00

(54) **Implant device with placement indicia**
Implantatsvorrichtung mit Platzierungshinweisen
Implant doté de repères facilitant son placement

(30) Priority: 19.06.2006 US 455345
(43) Date of publication of application: 26.12.2007
(73) Proprietor: DePuy Products, Inc., Warsaw, IN 46581 (US)
(72) Inventor: Sanders, Roy W., Tampa, FL 33606 (US); Myerson, Mark S., Baltimore, MD 21202 (US); Prasad, Priya R., Miami, FL 33131 (US)
(74) Representative: Belcher, Simon James

(56) References cited:
- EP-A- 1 477 124
- WO-A-2004/045455
- WO-A-2007/035892
- WO-A-2007/127994
- FR-A- 2 405 062
- US-A1- 2006 085 000
- US-A1- 2006 106 387

## Description

This invention relates to the field of biomechanical implants secured to the bone and correct placement of such implants in the body.

Biomechanical implants are commonly used in surgical procedures. In the field of orthopaedics, implants are often secured to a bone to aid in healing, to prevent further damage, or even to completely replace portions of bone. Examples of such orthopaedic implants include plates, pins, rods, wires, and screws secured to the bone. Another example of an orthopaedic implant is a prosthetic device, such as a prosthetic knee, that replaces all or part of a bone structure and/or its surrounding tissue.

When a surgeon places an orthopaedic implant in a patient, there is typically an exact placement location that is optimal or otherwise preferred for the implant. There is also typically an optimal or preferred orientation and alignment for the implant. Correct placement and orientation of an implant with respect to the bone is important, as an implant that is properly positioned within a patient will perform better and cause fewer problems than an improperly positioned implant.

Under current practice, optimal placement and orientation information for an implant is typically communicated to the surgeon through surgical technique literature provided with the implant. In particular, when an implant device is sold or otherwise delivered to a surgeon, a product manual or brochure is commonly included with the implant describing the product in some detail and recommending an optimal placement location and orientation for the implant with respect to the bone. The surgeon is expected to review this information prior to surgery, thus providing the surgeon with a good understanding of the implant and its optimal placement location and orientation.

Unfortunately, many surgeons do not properly review product literature before proceeding with an implant surgery. Failure to review product literature may occur for numerous reasons. When a surgeon fails to review product literature, the surgeon may not fully understand preferred placement of the implant, and the implant may not be placed in the optimal location in the patient. Accordingly, it would be advantageous to provide implant products that suggest or otherwise communicate correct product placement to the surgeon even if the surgeon fails to read the product literature.

WO-A-2004/045455 discloses a bone plate which has openings extending through it in which bone screws can be received, and a bridge region between two of the openings which is narrowed so that the plate deforms preferentially in this region.

US-2006/0085000-A1 discloses a bone plate which has alignment indicia as defined in the preamble of appended claim 1.

The present invention provides a bone plate as defined in claim 1.

Examples of anatomical features that may be found in a patient's body, which can be aligned with the bone plate, include joint lines, bone fractures, as well as tendon or ligament attachment sites.

The bone plate has a contoured perimeter. Alignment indicia are formed in the perimeter between the first end portion and the second end portion. The alignment indicia are configured to indicate proper positioning of the bone plate on the at least one bone with respect to the anatomical feature. The alignment indicia may comprise, for example, protrusions or indentations formed in the perimeter of the bone plate. However, the alignment indicia formed in the perimeter of the bone plate are not associated with any of the plurality of fixation holes. Accordingly, the alignment indicia are not associated with protrusions in the perimeter that are formed around one of the plurality of fixation holes. In addition, the alignment indicia are not associated with indentations in the perimeter that result from two adjacent protrusions in the perimeter formed around two of the plurality of fixation holes.

Additional alignment indicia, such as etching, may also be provided on the bone plate. The etching may comprise text or other symbols that assist the surgeon with proper orientation and alignment of the bone plate on the bone.

The bone plate is generally comprised of a bio-compatible material that is also radio opaque. Use of such material allows the surgeon to confirm proper placement of the bone plate in the patient using fluoroscopy or other x-ray imaging procedures. In alternative embodiments other materials may be used that are compatible with other imaging procedures.

Use of the bone plate with alignment information as described herein has the advantage of helping to ensure proper placement of the bone plate in the patient. In particular, the alignment indicia provided on the bone plate provide the surgeon with valuable placement information that is readily available during the implantation procedure. During the surgical procedure, the surgeon places the bone contacting structure on the at least one bone, aligning the alignment indicia on the bone plate with an anatomical feature related to the at least one bone. During and/or after surgery, the surgeon may confirm proper placement of the bone plate by confirming alignment of the alignment indicia with the at least one anatomical feature using an imaging procedure such as fluoroscopy.

The device of the invention can be used in a method of attaching an implant to at least one bone in a body, the method comprising:
a) providing an implant comprising a bone contacting structure defining a perimeter, in which alignment indicia is formed along the perimeter of the bone contacting structure;
b) placing the bone contacting structure on the at least one bone;
c) aligning the alignment indicia with an anatomical feature related to the at least one bone; and
d) confirming the alignment of the alignment indicia with the at least one anatomical feature using an imaging procedure.

Embodiments of the invention will now be described by way of example with reference to the accompanying drawings, in which:
FIG. 1 shows an exemplary implant including indicia to indicate proper placement and orientation of the implant relative to a joint line; and
FIG. 2 shows an exemplary implant including indicia to indicate proper placement and orientation of the implant relative to a bone fracture.

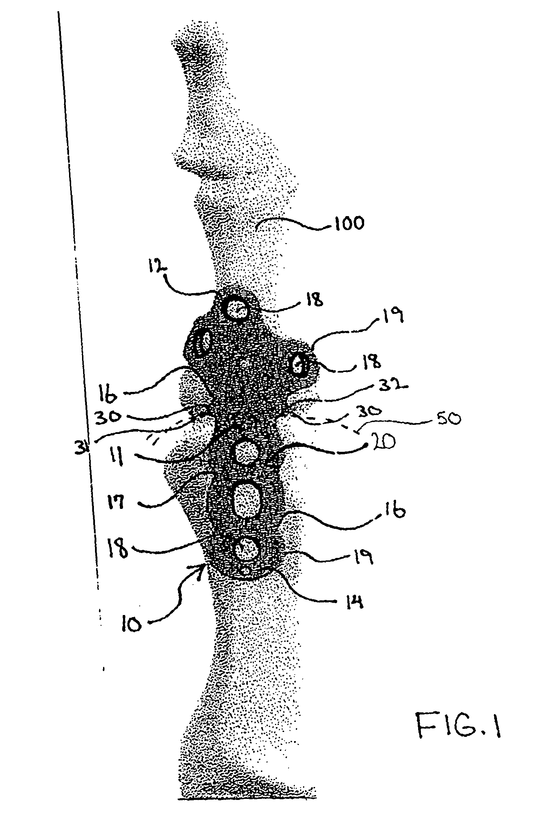

Referring to the drawings, FIG. 1 show an implant device in the form of a bone fixation device, particularly a bone plate 10, is shown having placement indicia formed in it. The bone plate 10 is shown positioned on a plurality of bones 100, being the metatarsal-phalangeal joint in the toe of a human.

The bone plate 10 is generally elongated in shape and includes a main body 11 bordered by a perimeter 16. The perimeter 16 defines the overall shape of the bone plate 10. The main body 11 includes a plurality of fixation holes 20 configured to receive screws, bolts, posts, or other fixation members designed to seat in the bone and secure the bone plate 10 to the bone.

The bone plate 10 generally includes two opposing surfaces, including a bone contacting surface (not shown) and an outward facing surface 20. The bone contacting surface is designed for placement against the bone. Accordingly, the bone contacting surface may be slightly concave relative to a longitudinal midline, allowing the bone contacting surface to conform to the bone while providing a greater surface area for actual contact with the bone 100. The outward facing surface 20 is opposite the bone contacting surface and faces away from the bone 100. The screws or other fixation devices are inserted through the holes 18 in the bone plate 10 and into the bone 100. When the screws are fixed to the bone, they secure the bone plate in place against the bone.

With continued reference to FIG. 1, the main body 11 of the bone plate has a first end 12 and an opposite second end 14. Several holes 18 are located in the first and second ends of the main body. The holes 18 in the first end 12 of the main body 11 are used in conjunction with screws or other fixation devices to secure the first end 12 to the bone 100. Likewise, the holes 18 in the second end 14 of the main body 11 are used in conjunction with screws or other fixation devices to secure the second end 14 to the bone 100.

The perimeter 16 of the bone plate 10 includes several contours and rounded bump out portions. Contours in the perimeter 16 follow the general shape of the surface 20 of the plate 10. The rounded bump out portions 19 are provided along the perimeter sides of each of the plurality of holes 18. These rounded bump out portions 19 generally provide additional plate area to strengthen the plate where the holes 18 are located. In particular, the bump out portions 19 provide additional plate material around the holes, and this additional plate material acts to resist bending of the plate near the holes.

Alignment indicia 30 are provided between the first end 12 and the second end 14 of the bone plate 10. In the embodiment of FIG. 1, the alignment indicia 30 comprise two protrusions extending from the main body 11 of the end plate and situated between the first end 12 and the second end 14 of the end plate. The alignment indicia 30 form contours along the perimeter 16 of the bone plate 10. In the embodiment of FIG.1, the alignment indicia in the perimeter 16 form two protrusions 31 and 32. No holes are located in the main body between these protrusions 31, 32. In other words, the central portion of the plate 10 which extends between the opposing alignment indicia 30 does not include a hole 18. This is not the case with the bump out portions 19, as the portion of the plate between opposing bump out portions 19 will cross a hole 18.

The alignment indicia 30 provided in the perimeter 16 of the plate 10 are not associated with any of the holes 18 in the plate. In particular, the alignment indicia 30 are separate from the bump out portions 19 which are each formed around one of the plurality of holes 18. Also, the alignment indicia 30 do not result from a contour in the perimeter that is related to the holes, such as an indentation 17 in the perimeter resulting from two adjacent bump out portions 19 formed around two of the holes 18.

The alignment indicia 30 are configured for alignment with an anatomical feature on a patient's body. For example, with the bone plate 10 disclosed in FIG. 1, the alignment indicia 30 are configured for alignment with a joint line 50. The joint line 50 is shown as a slightly curved line in FIG. 1 extending along the metatarsal-phalangeal joint. As shown in FIG. 1, when the implant is properly placed on the bones, the protrusions 31, 32 are positioned along the joint line 50. In alternative embodiments, the alignment indicia 30 are configured for alignment with other anatomical features, including those examples discussed in further detail below.

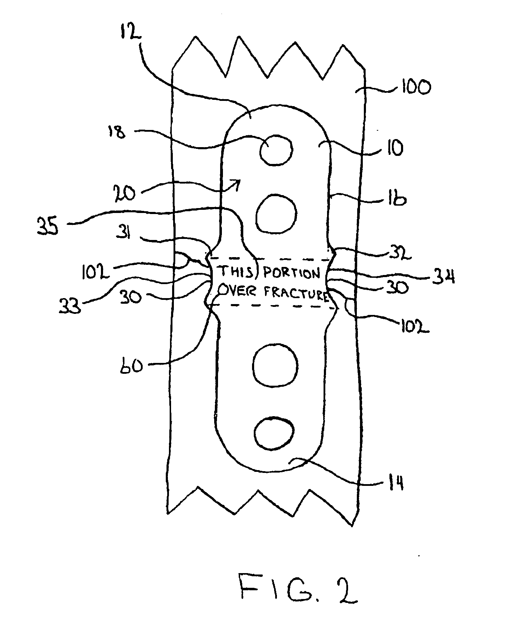

One example of another anatomical feature that the alignment indicia 30 may be configured for alignment with is a bone fracture. An example of a bone plate 10 positioned on a bone 100 with a fracture 102 is disclosed in FIG. 2. In this embodiment, the alignment indicia 30 formed in the perimeter 16 of the bone plate include protrusions 31 and 32. The protrusions 31 and 32 of FIG. 2 cause the perimeter to bulge outward. However, the shape of the protrusions also include slight indentations 33 and 34 that cause the perimeter to bend inwardly. The indentations 33 and 34 in the protrusions 31 and 32 are provided to indicate proper alignment of the bone plate 10 with the fracture 102 in the bone 100. In particular, the bone plate 10 is configured such that the portion of the bone plate 35 between the indentations 33 and 34 should cover a portion of the fracture, and the fracture line 102 should appear to extend outward from the indentations 33 and 34.

The alignment indicia 30 formed in the perimeter 16 of the bone plate 10 may have various other shapes and sizes other than the formations shown herein. Accordingly, the alignment indicia 30 formed in the perimeter may comprise is not limited to the protrusions 31, 32 or indentations 33, 34 such as those shown in FIGs. 1 and 2.

In addition to alignment indicia 30 formed along the perimeter of the bone plate 10, the alignment indicia may also be provided in additional locations. For example, additional alignment indicia may comprise etched symbols or text in the bone plate. Exemplary etching is shown in FIG. 2, where text 60 is shown etched in the bone plate 10. The etched text 60 provides instructions to the surgeon indicating where the bone plate should be positioned relative to the anatomical feature. In FIG. 2, the text 60 instructs the surgeon to locate the bone plate on the bone such a central portion 35 of the bone plate 10 covers a substantial portion of the fracture. With this orientation, the fracture line 102 in the bone 100 is aligned with the indentations 33 and 34 formed in the perimeter 16 of the bone plate 10. Accordingly the alignment indicia 30 cover a portion of the fracture, and the fracture line 102 extends outward from the indentations 33 and 34. While the indentations 33 and 34 serve as perimeter alignment indicia, the etching serves as additional alignment indicia to assist the surgeon in making an optimal placement of the bone plate 10 on the bone 100. If etching is provided as additional alignment indicia, the etching is typically relatively shallow to avoid compromising the structural integrity of the implant.

The bone plate 10 is generally comprised of a rigid biocompatible material, such as CoCr, titanium, or other material. In one embodiment, the biocompatible material is radio-opaque such that the plate is detectable by radiography or fluoroscopy. Use of such materials allows the surgeon to confirm correct placement of the bone plate 10 with respect to the designated anatomical features during or following surgery. In particular, alignment indicia along the perimeter of the plate may be clearly seen in the patient using a radiograph, fluoroscope or other x-ray device. Visual confirmation that such alignment indicia is properly aligned with an anatomical feature also showing up on the x-ray device, provides assurance that the bone plate is properly implanted in the patient. It is also envisaged that other biocompatible materials may be used that compliment different imaging procedures available to the surgeon, such as magnetic resonance imaging or ultrasound.

The rigidity and resiliency of the material used for the bone plate may vary, depending upon the application. For example, if the bone plate is used in association with a fracture, the material may be more rigid such that the bone plate prevents movement of the bone along the fracture. As another example, if the implant is provided along a joint, the implant may be comprised of more flexible material, allowing some limited movement of the joint.

In operation, the bone plate or other implant device is provided to the surgeon along with instructions for implantation of the bone plate. Preferably, the surgeon will read the instructions to obtain an understanding of proper placement of the implant within the patient. However, regardless of whether the surgeon reads the provided instructions, the alignment indicia provided on the implant will assist the surgeon with proper alignment of the implant. In particular, in order to properly orient and align the implant in the patient, the surgeon simply aligns the alignment indicia with an anatomical feature of the patient. Such alignment may include, for example, pointing or aligning one or more protrusions or indentations provided along the perimeter of the implant toward an anatomical feature on the patient, such as a joint line or a fracture. Etching may be provided on the implant to further assist the surgeon with proper alignment of the implant. In lieu of etching, a sticker or piece of paper that includes implant instructions related to the alignment indicia may be provided on the implant or packaged with the implant.

After using the alignment indicia to properly place the implant against one or more bones, the surgeon secures the implant to the bone. For example, if the bone plate 10 of FIGs. 1 or 2 is implanted in a patient, the surgeon uses a jig or other device to temporarily secure the bone plate to the bone after the alignment indicia is aligned with the anatomical feature. Next, the surgeon drills holes in the bone aligned with the holes 18 of the bone plate 10. The surgeon then inserts bone screws that extend through the holes 18 and into the bone. Thus, bone screws are provided on opposite sides of the bone plate with respect to the anatomical feature used for alignment.

Before actually fixing the implant to the patient, an imaging procedure may be used to confirm proper placement of the implant with respect to the anatomical feature of the patient. For example, fluoroscopy may be used to confirm that the bone plate is correctly placed on the bone relative to a bone fracture, joint line, tendon or ligament attachment site, or other anatomical feature. Because the alignment indicia is provided along the perimeter of the implant, the alignment indicia can be clearly seen during fluoroscopy and other imaging procedures. If the implant is properly positioned in the patient, the surgeon proceeds with fixing the implant to the patient. Imaging procedures may also be used following surgery to confirm that the implant remains in a correct or optimal position in the patient with respect to a particular anatomic feature of the patient.

## Claims

1. A bone plate (10) configured for attachment to at least one bone located in a body, in which an anatomical feature exists in the body related to the at least one bone, the bone plate comprising:
a) a first end portion (12) configured for attachment to the at least one bone on a first side of the anatomical feature, having a plurality of fixation holes (18) formed in it, and
b) a second end portion (14) configured for attachment to the at least one bone on a second side of the anatomical feature, having a plurality of fixation holes (18) formed in it, in which the second side is opposite the first side,
wherein the bone plate has alignment indicia (30) in a central portion of the plate between the first and second end portions, which central portion of the plate does not have any holes formed in it, wherein the alignment indicia are configured to indicate proper positioning of the bone plate on the at least one bone with respect to the anatomical feature,
**characterised in that** the indicia comprise protrusions (31, 32) extending opposite to one another from opposite sides of the bone plate in the central region.

2. The bone plate of claim 1 in which the alignment indicia comprise two protrusions (31, 32) on each side of the plate.

3. The bone plate of claim 1 which includes etching (60) on the bone plate (10) related to the alignment indicia (30).

4. The bone plate of claim 3 in which the etching (60) comprises text or at least one symbol.

## Patentansprüche

1. Knochenplatte (10), die zur Befestigung an wenigstens einem Knochen, der sich in einem Körper befindet, ausgelegt ist, wobei ein anatomisches Merkmal in dem Körper vorhanden ist, das einen Bezug zu dem wenigstens einen Knochen hat, wobei die Knochenplatte aufweist:
a) einen ersten Endbereich (12), der zur Befestigung an dem wenigstens einen Knochen auf einer ersten Seite des anatomischen Merkmals ausgelegt ist, mit einer Vielzahl von Fixierlöchern (18), die darin gebildet sind, und
b) einen zweiten Endbereich (14), der zur Befestigung an dem wenigstens einen Knochen auf einer zweiten Seite des anatomischen Merkmals ausgelegt ist, mit einer Vielzahl von Fixierlöchern (18), die darin gebildet sind, wobei die zweite Seite der ersten Seite gegenüberliegt,
wobei die Knochenplatte Ausrichtemerkmale (30) in einem mittigen Bereich der Platte zwischen dem ersten und dem zweiten Endbereich hat, wobei in dem mittigen Bereich der Platte keinerlei Löcher gebildet sind, wobei die Ausrichtemerkmale so gestaltet sind, dass sie das richtige Positionieren der Knochenplatte auf dem wenigstens einen Knochen mit Bezug auf das anatomische Merkmal angeben,
**dadurch gekennzeichnet, dass** die Merkmale Vorsprünge (31, 32) aufweisen, die sich von gegenüberliegenden Seiten der Knochenplatte in dem mittigen Bereich einander gegenüberstehend erstrecken.

2. Knochenplatte nach Anspruch 1, bei der die Ausrichtemerkmale zwei Vorsprünge (31, 32) auf jeder Seite der Platte aufweisen.

3. Knochenplatte nach Anspruch 1, die eine Ätzung (60) auf der Knochenplatte (10) mit Bezug auf die Ausrichtemerkmale (30) umfassen.

4. Knochenplatte nach Anspruch 1, bei der die Ätzung (60) Text oder wenigstens ein Symbol aufweist.

## Revendications

1. Plaque osseuse (10) configurée pour la fixation à au moins un os situé dans un corps, dans lequel il existe une caractéristique anatomique dans le corps par rapport au au moins un os, la plaque osseuse comprenant :
a) une première partie d'extrémité (12) configurée pour la fixation au au moins un os sur un premier côté de la caractéristique anatomique, ayant une pluralité de trous de fixation (18) formée dans cette dernière, et
b) une seconde partie d'extrémité (14) configurée pour la fixation au au moins un os sur un second côté de la caractéristique anatomique, ayant une pluralité de trous de fixation (18) formée à l'intérieur de cette dernière, dans laquelle le second côté est opposé au premier côté,
dans laquelle la plaque osseuse a des repères d'alignement (30) dans une partie centrale de la plaque entre les première et seconde parties d'extrémité, laquelle partie centrale de la plaque n'a pas de trou formé à l'intérieur de celle-ci, dans laquelle les repères d'alignement sont configurés pour indiquer le bon positionnement de la plaque osseuse sur le au moins un os par rapport à la caractéristique anatomique,
**caractérisé en ce que** les repères comprennent des saillies (31, 32) s'étendant à l'opposé les unes des autres à partir des côtés opposés à la plaque osseuse dans la région centrale.

2. Plaque osseuse selon la revendication 1, dans laquelle les repères d'alignement comprennent deux saillies (31, 32) de chaque côté de la plaque.

3. Plaque osseuse selon la revendication 1, qui comprend une gravure (60) sur la plaque osseuse (10) par rapport aux repères d'alignement (30).

4. Plaque osseuse selon la revendication 3, dans laquelle la gravure (60) comprend du texte ou au moins un symbole.
